# EUROPEAN PATENT APPLICATION

(11) **EP 4 830 672 A1**
(43) Date of publication of application: **28.10.2026**
(21) Application number: 24912306.8
(22) Date of filing: 09.12.2024
(51) Int. Cl.: A61F 2/852

(54) **STENT AND METHOD FOR IMAGING STENT**

(30) Priority: 25.12.2023 JP 2023218655
(71) Applicant: T.G. Medical Inc., Tokyo 103-0023 (JP)
(72) Inventor: MIKI Kohei, Tokyo 103-0023 (JP)
(74) Representative: Appleyard Lees IP LLP
(86) International application number: PCT/JP2024/043337
(87) International publication number: WO 2025/142417

(57) **Abstract**

Provided are a stent that makes it possible to distinguish and identify a marker provided on a wire from other markers, and a method for imaging the stent. A stent 1 is inserted into and delivered through a catheter C, and is used for an application of expanding a stenotic area in a biological lumen. The stent 1 comprises a wire 2 and stent bodies 10, 20 connected to the distal side of the wire 2. The stent 1 further comprises a first wire marker part 31 provided on the wire 2, and a second wire marker part 32 provided on the wire 2 and located distal to the first marker 31 with a space therebetween in the axial direction of the stent 1.

## Description

### TECHNICAL FIELD

The present disclosure relates to a stent and a method for capturing an image of a stent.

### BACKGROUND ART

A device which uses a stent for removing and retrieving a thrombus formed in a blood vessel (hereinafter, also referred to as a thrombectomy stent) has been known (see Patent Document 1). This thrombectomy stent is configured in a form suitable for thrombus retrieval, and in order to entangle the thrombus in the stent to retrieve the thrombus, the stent expanded in the blood vessel is pulled toward a catheter side (a proximal side), and in this manner, the stent is retrieved together with the thrombus.

On the other hand, when stenosis occurs in a living body lumen with a lumen structure, such as a blood vessel, a trachea, and an intestine, it has been examined to use a stent (hereinafter, also referred to as a stenosis dilation stent) in order to ensure the patency of a lesion area by expanding a lumen of a stenotic part. The stenosis dilation stent is configured to be suitable for expanding the stenotic part, and contacts an inner wall of the living body lumen at a predetermined expansion force, so that the stent is placed therein. Thus, when a position of the stenosis dilation stent is unintentionally moved (hereinafter, unintentionally moved is also referred to as "accidentally moved") while the stenosis dilation stent is in contact with the inner wall of the living body lumen in a process of deploying the stent or a process of sheathing the stent in the catheter, the inner wall of the living body lumen may be damaged. Consequently, it is important to operate the stenosis dilation stent so that a position of the stenosis dilation stent is not accidentally moved in a state in which a stent body is deployed.

Further, a radiopaque member (so-called marker) which is highly opaque to radiation such as X-rays is provided in the stent in order to check a position or the like of the stent inside the lumen structure when the stent is arranged in the lumen structure (see Patent Document 2). With such a stent, it is possible to visually recognize the radiopaque member provided in the stent by applying radiation and improve the operability of the stent, accordingly.

A wire is connected to the stenosis dilation stent on a proximal side with respect to a stent body. When it is desired to change the position of the stent body, the wire is moved toward the proximal side or the distal side. Consequently, when a marker is also provided on this wire, it is considered that this makes it possible to check whether a force for accidentally moving the stent body portion is applied.

Moreover, it may be possible to check whether the stent body portion is in a state of being sheathed in the catheter (a diameter-narrowed state) or in a state of being completely removed from the catheter (a diameter-expanded state), on the basis of a relative position relationship between the marker provided on the wire and the marker provided on the catheter.

### Citation List

### Patent Documents

Patent Document 1: Japanese Unexamined Patent Application, Publication No. 2021-106928
Patent Document 2: Japanese Unexamined Patent Application, Publication No. 2014-171893

### DISCLOSURE OF THE INVENTION

### Problems to be Solved by the Invention

However, when the marker to be provided on the wire is provided in a similar form to the marker provided on the stent body portion as in Patent Document 2, it may be difficult to distinguish and recognize the marker for the wire and the marker for the stent body.

A problem of the present disclosure is to provide a stent in which a marker provided on a wire can be checked by being distinguished from the other markers, and a method for capturing an image of a stent.

### Means for Solving the Problems

The present disclosure addresses the above-described problem by the following solution. Note that for the sake of easy understanding, the present disclosure will be described with reference numerals corresponding to an embodiment, but the disclosure is not limited thereto.

A first aspect of the present disclosure is directed to a stent (1, 1B) that is inserted into and delivered through a catheter (C) and is used for an application to expand a stenotic part in a living body lumen, the stent (1, 1B) including: a wire (2); a stent body (10, 20) that is connected to a distal side of the wire (2); a first wire marker (31, 31B) provided on the wire (2); and a second wire marker (32) provided on the wire (2) to be distanced from the first wire marker (31, 31B) toward a distal side in an axial direction of the stent (1, 1B).

A second aspect of the present disclosure is directed to a stent (1, 1B) that is inserted into and delivered through a catheter (C) and is used for an application to expand a stenotic part in a living body lumen, the stent (1, 1B) including: a wire (2); a first stent body (10) that is connected to a distal side of the wire (2) and in which a plurality of first cells (12) including a plurality of first struts (11) are arrayed in a circumferential direction and in a central axis direction; a second stent body (20) in which a plurality of second cells (22) including a plurality of second struts (21) are arrayed in the circumferential direction and the central axis direction and at least a region that expands the living body lumen is inserted into the first stent body (10); a first wire marker (31, 31B) provided on the wire (2); and a second wire marker (32) provided on the wire (2) to be distanced from the first marker (31, 31B) toward a distal side in an axial direction of the stent (1, 1B).

According to a third aspect of the present disclosure, in the stent (1, 1B) according to the first or second aspect, the first wire marker (31, 31B) is configured to have a longer length than the second wire marker (32).

According to a fourth aspect of the present disclosure, in the stent (1B) according to the third aspect, the first wire marker (31B) is provided toward a proximal side of the wire (2) continuously or in multiple parts that are distanced apart from each other, in a range of 20 mm or more from a position of a distal side end portion of the first wire marker toward a proximal side.

A fifth aspect of the present disclosure is directed to a method for capturing an image of the stent (1, 1B) according to the aspect 1 or 2 in a living body lumen, the method including: capturing an image of the first wire marker (31, 31B) and the second wire marker (32) in a diameter-narrowed state of the stent (1, 1B) at a position where the stent (1, 1B) is to be expanded in the living body lumen; and capturing an image of the first wire marker (31, 31B) and the second wire marker (32) in a diameter-expanded state of the stent (1, 1B).

According to a sixth aspect of the present disclosure, in the method for capturing an image of the stent (1, 1B) according to the fifth aspect, a catheter marker (CM) which is highly opaque to radiation is provided at a distal end of the catheter (C) or in a vicinity of the distal end, in capturing the image in the diameter-narrowed state of the stent (1, 1B), an image of the catheter marker (CM) is captured together with the first wire marker (31, 31B) and the second wire marker (32) in a state in which the catheter marker (CM) is on the distal side with respect to the second wire marker (32), and in capturing the image in the diameter-expanded state of the stent (1, 1B), an image of the catheter marker (CM) is captured together with the first wire marker (31, 31B) and the second wire marker (32) in a state in which the catheter marker (CM) overlaps with a separation portion (33) located between the first wire marker (31, 31B) and the second wire marker (32) or in a state in which the catheter marker (CM) is on a proximal side with respect to the separation portion (33).

According to a seventh aspect of the present disclosure, in the method for capturing an image of the stent (1, 1B) according to sixth aspect, when the stent (1, 1B) is removed from the catheter (C) and expanded, after capturing the image in the diameter-narrowed state of the stent (1, 1B) is performed, the catheter (C) is moved toward the proximal side while maintaining a position of the stent (1, 1B), and capturing the image in the diameter-expanded state of the stent (1, 1B) is performed, and then, when the stent (1, 1B) is sheathed in the catheter (C), the catheter (C) is moved toward the distal side while maintaining the position of the stent (1, 1B) and capturing an image in the diameter-narrowed state of the stent (1, 1B) is performed again.

An eighth aspect of the present disclosure is directed to a method for expanding a stenotic part in a living body lumen using a stent (1, 1B) that is inserted into and delivered through a catheter (C) and is used for an application to expand a stenotic part in a living body lumen, the stent (1, 1B) including: a wire (2); a stent body (10, 20) that is connected to a distal side of the wire (2); a first wire marker (31, 31B) provided on the wire (2); and a second wire marker (32) provided on the wire (2) to be distanced from the first wire marker (31, 31B) toward a distal side in an axial direction of the stent (1, 1B), the method including: delivering the stent (1, 1B) sheathed in the catheter (C) in a diameter-narrowed state together with the catheter (C), through the living body lumen, to a position (P) where the living body lumen is to be expanded; retracting the catheter (C) to a proximal side, starting to expand the stent (1, 1B) from a distal side, and capturing an X-ray transmission video of the first wire marker (31, 31B) and the second wire marker (32) while moving the stent; completing expansion of the stent (1, 1B); and resheathing the expanded stent (1, 1B) in the catheter (C).

A ninth aspect of the present disclosure is directed to a method for expanding a stenotic part in a living body lumen using a stent (1, 1B), the stent (1, 1B) including: a wire (2); a first stent body (10) that is connected to a distal side of the wire (2) and in which a plurality of first cells (12) including a plurality of first struts (11) are arrayed in a circumferential direction and in a central axis direction; a second stent body (20) in which a plurality of second cells (22) including a plurality of second struts (21) are continuous in the circumferential direction and the central axis direction and at least a region that expands the living body lumen is inserted into the first stent body (10); a first wire marker (31, 31B) provided on the wire (2); and a second wire marker (32) provided on the wire (2) to be distanced from the first wire marker (31, 31B) toward a distal side in an axial direction of the stent (1, 1B), the method including: delivering the stent (1, 1B) sheathed in the catheter (C) in a diameter-narrowed state together with the catheter (C), through the living body lumen, to a position (P) where the living body lumen is to be expanded; retracting the catheter (C) to a proximal side, starting to expand the stent (1, 1B) from a distal side, and capturing an X-ray transmission video of the first wire marker (31, 31B) and the second wire marker (32) while moving the stent; completing expansion of the stent (1, 1B); and resheathing the expanded stent (1, 1B) in the catheter (C).

### Effects of the Invention

According to the present disclosure, there can be provided a stent in which a marker provided on a wire can be checked by being distinguished from the other markers, and a method for capturing an image of a stent.

### BRIEF DESCRIPTION OF THE DRAWINGS

FIG. 1 is a side view of a stent 1 of a first embodiment;
FIG. 2 is a perspective view of the stent 1 shown in FIG. 1;
FIG. 3A is a virtual development view of a part of a first stent body 10 of the first embodiment;
FIG. 3B is a virtual development view of a part of a second stent body 20 of the first embodiment;
FIG. 3C is a virtual development view of a part of the stent 1 of the first embodiment;
FIG. 4A is a diagram for describing an outer diameter D1 of a first stent body 10 alone;
FIG. 4B is a diagram for describing an outer diameter D2 of a second stent body 20 alone;
FIG. 5 is a diagram showing a process of inserting the second stent body 20 into the first stent body 10;
FIG. 6 is a sectional view in which the stent 1 is cut at a position of an arrow s1-s1 in FIG. 1;
FIG. 7 is a diagram for describing an internal state when the stent 1 is bent;
FIG. 8 is an exploded and enlarged diagram of the vicinity of a distal LD2 side end portion of a wire 2;
FIG. 9 is a diagram schematically showing an example of an image captured in capturing an image of a second stent marker 121 in a diameter-narrowed state of the stent 1 at a position where the stent 1 is to be expanded in the living body lumen;
FIG. 10 is a diagram schematically showing an example of an image captured in capturing an image of the second stent marker 121 in an intermediate state while transitioning from a diameter-narrowed state to a diameter-expanded state of the stent 1 at the position where the stent 1 is to be expanded in the living body lumen;
FIG. 11 is a diagram schematically showing an example of an image captured in capturing an image of the second stent marker 121 in a diameter-expanded state of the stent 1 at the position where the stent 1 is expanded in the living body lumen;
FIG. 12 is a diagram showing an example of an image captured in capturing an image in a diameter-narrowed state of the stent;
FIG. 13 is a diagram showing an example of an image captured in capturing an image while moving the stent;
FIG. 14 is a diagram showing an example of an image captured in capturing an image in a diameter-expanded state of the stent;
FIG. 15 is a schematic side view of a stent 1B of a second embodiment;
FIG. 16 is a schematic diagram showing an ideal state in which an extra force is not applied to the wire 2 in a state in which the stent 1B of the second embodiment is expanded;
FIG. 17 is a schematic diagram showing a state in which a force with which the wire 2 is pulled toward a proximal LD1 side is applied in a state in which the stent 1B of the second embodiment is expanded;
FIG. 18 is a schematic diagram showing a state in which a force with which the wire 2 is pushed toward a distal LD2 side is applied in a state in which the stent 1B of the second embodiment is expanded; and
FIG. 19 is a schematic diagram showing a state in which a force with which the wire 2 is pushed toward a distal LD2 side is applied in a state in which the stent 1B of the second embodiment is expanded.

### PREFERRED MODE FOR CARRYING OUT THE INVENTION

Hereinafter, embodiments of a stent will be described. Note that each of the drawings attached to the present specification shows a schematic view and the shape, scale, longitudinal-lateral dimensional ratio, etc. are changed or exaggerated as compared to actual shape, scale, longitudinal-lateral dimensional ratio, etc. Moreover, in the drawings, hatching showing the cross-section of members may be omitted where appropriate.

In the present specification etc., terms specifying shapes, geometric conditions, and the degrees thereof, such as "perpendicular" and "direction", include not only exact meanings of these terms, but also ranges taken as being substantially perpendicular and being substantially in a direction. In the present specification etc., a (proximal) side close to a practitioner will be referred to as a side LD1 (or a proximal LD1 side), and a (distal) side distant from the practitioner will be referred to as a side LD2 (or a distal LD2 side) in an axial direction (a longitudinal direction) LD. And a direction perpendicular to the axial direction LD will be referred to as a radial direction RD. Moreover, in the present specification etc., a direction in which cells are arrayed will be referred to as a circumferential direction OD. The circumferential direction may be not only the radial direction RD, but also a direction inclined with respect to the radial direction RD.

### (First Embodiment)

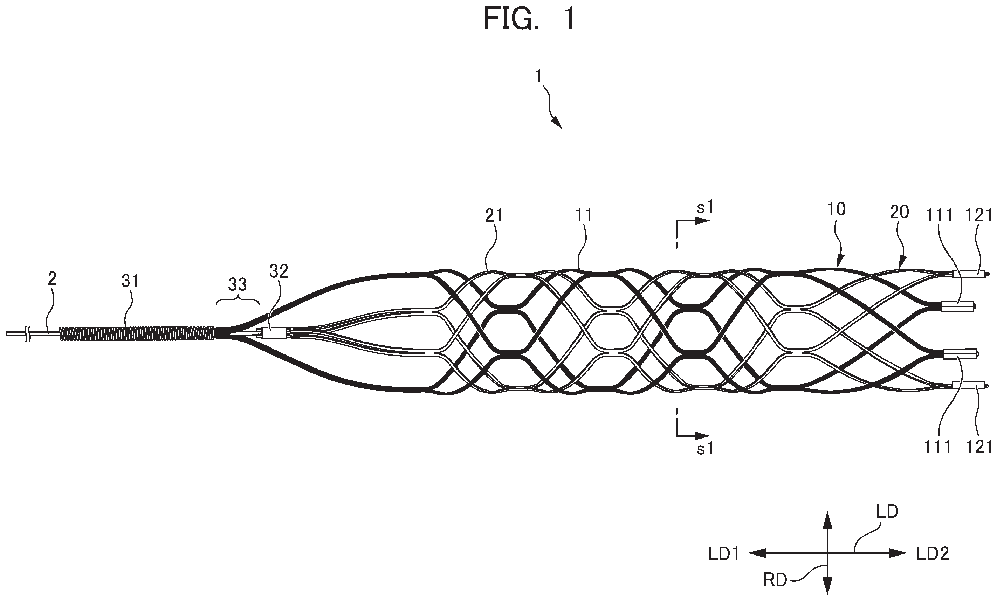

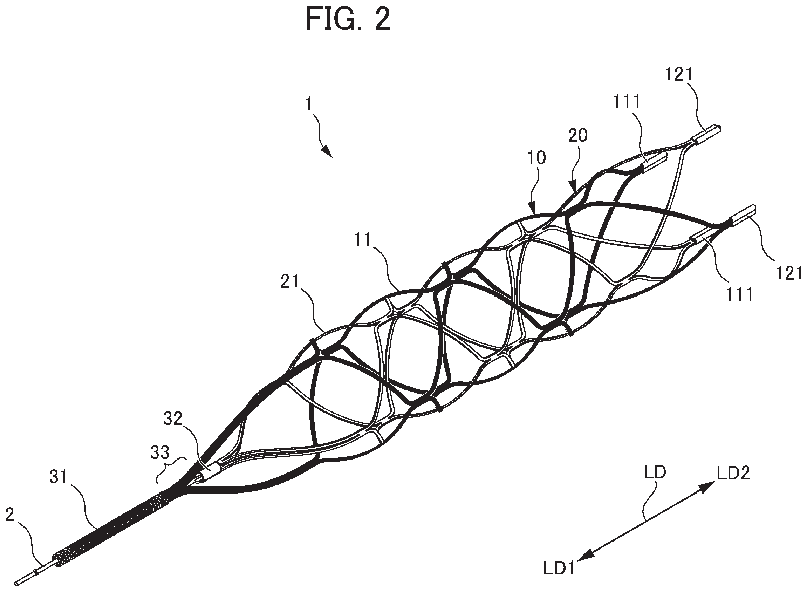

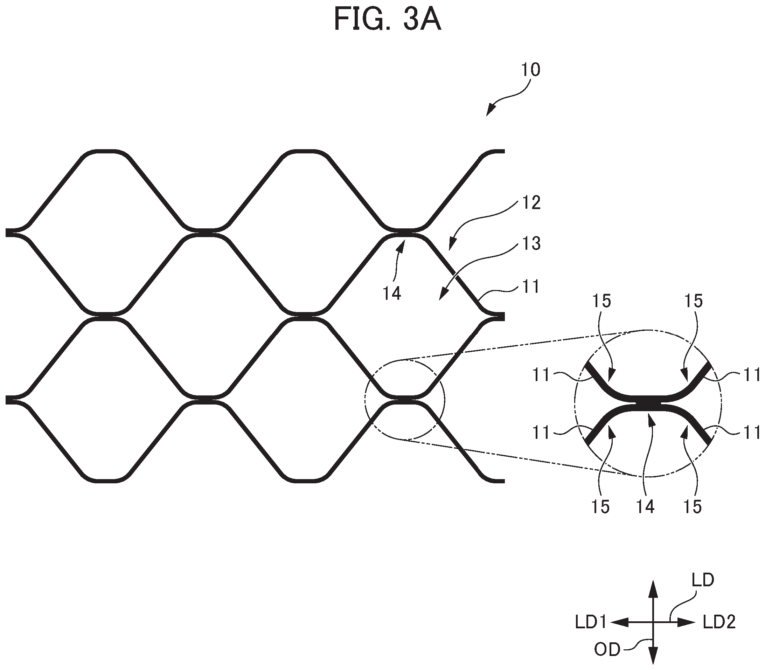

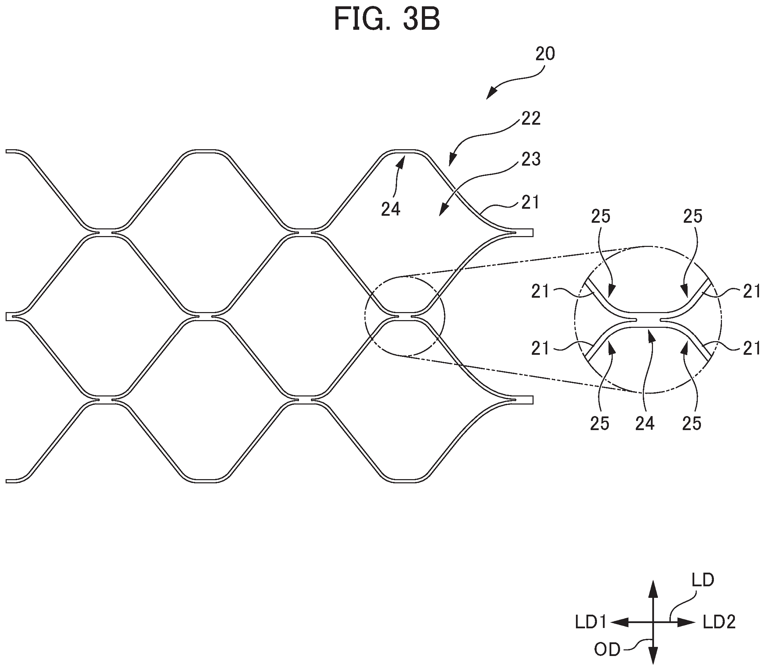

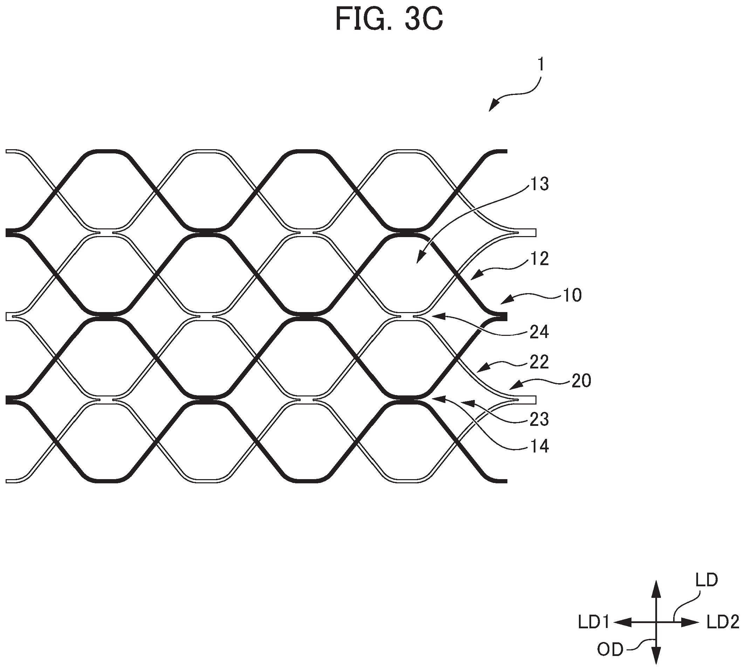

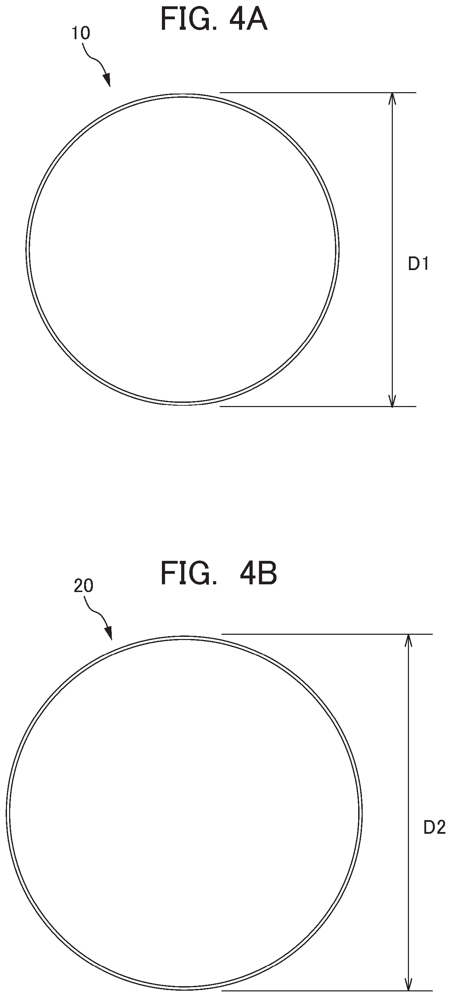

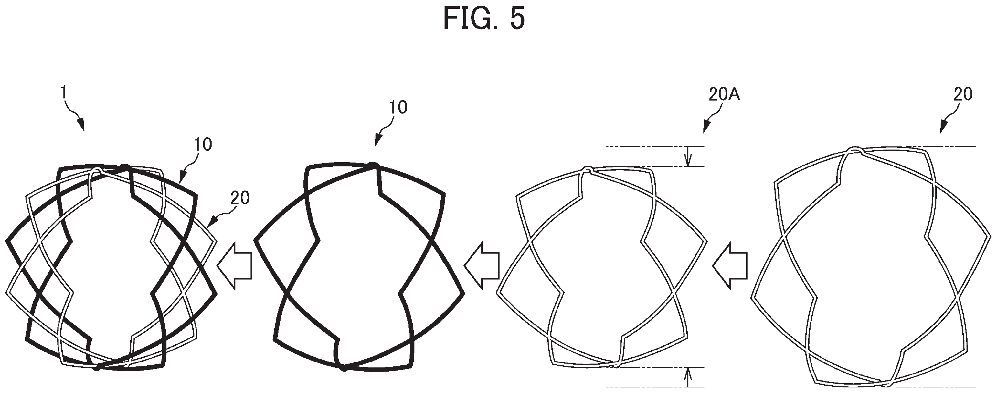

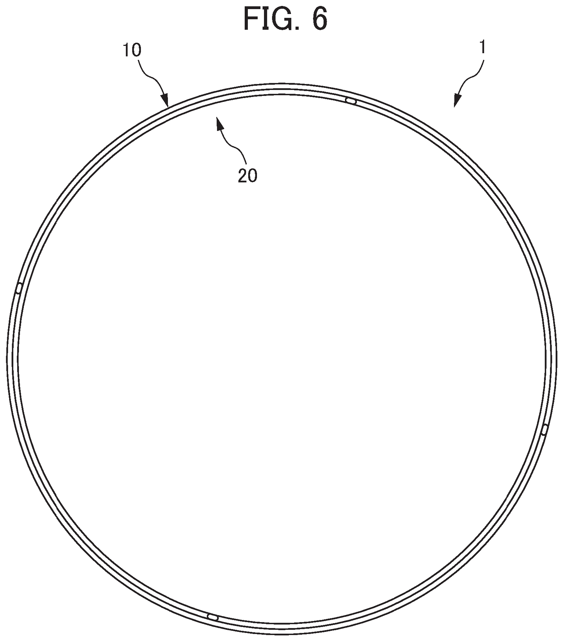

FIG. 1 is a side view of a stent 1 of a first embodiment. FIG. 2 is a perspective view of the stent 1 shown in FIG. 1. FIG. 3A is a virtual development view of a part of a first stent body 10 of the first embodiment. FIG. 3B is a virtual development view of a part of a second stent body 20 of the first embodiment. FIG. 3C is a virtual development view of a part of the stent 1 of the first embodiment. FIG. 4A is a diagram for describing an outer diameter D1 of a first stent body 10 alone. FIG. 4B is a diagram for describing an outer diameter D2 of a second stent body 20 alone. FIG. 5 is a diagram showing a process of inserting the second stent body 20 into the first stent body 10. FIG. 6 is a sectional view in which the stent 1 is cut at a position of an arrow s1-s1 in FIG. 1.

In the drawings, for the sake of easy distinguishing of the first stent body 10 and the second stent body 20, a strut of the first stent body 10 is indicated by black, and a strut of the second stent body 20 is indicated by white. Moreover, in the present specification etc., the "cell" indicates a portion surrounded by struts forming a stent. "Cells" may be identical in shape and size to each other or may be different in size from each other. The "strut" indicates a portion made of wire-like material. In the present specification etc., a cell opening will also be referred to as an "opening portion", and a portion where struts of adjacent cells are connected to or overlap with each other will also be referred to as an "overlapping portion".

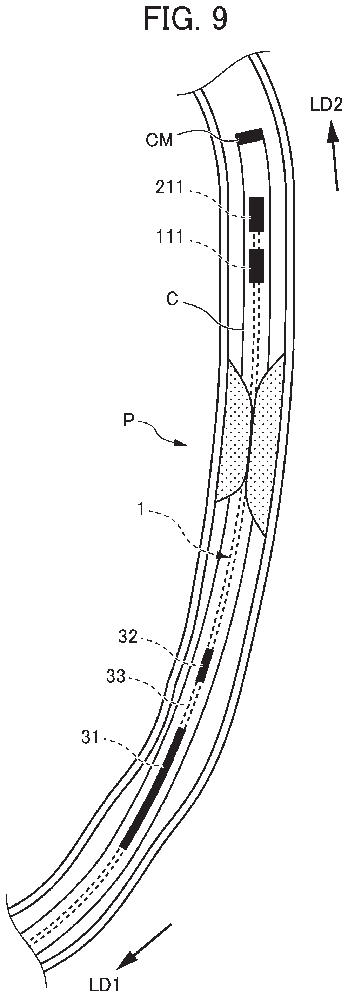

The stent 1 of the first embodiment may be used, for example, as follows: the stent 1 is sheathed (housed) in a catheter C (see FIGS. 9 to 11), pushed out of the catheter and expanded in a vascular lumen, such that the stent 1 expands a narrowed or occluded blood vessel. The stent 1 is used for an application to be retrieved from the body after having been temporarily placed in a blood vessel. Alternatively, the stent 1 may be used while remained in the blood vessel without being retrieved from the body. When the stent 1 is used for such an application, it may be preferable that a wire 2 and the stent 1 are configured to be separable from each other through the operation by a practitioner.

As shown in FIGS. 1 and 2, the stent 1 is in a substantially cylindrical shape in a diameter-expanded state. The stent 1 is in an elongated cylindrical shape in a diameter-narrowed state. The wire 2 is connected to a proximal LD1 side end portion. A method for connecting the proximal side end portion of the stent 1 and the wire 2 to each other is not particularly limited as long as the connection method is used for general medical equipment. Examples of the method may include welding, UV bonding, and silver solder infiltration.

The practitioner pushes or pulls the wire 2 via an operation unit (not shown) outside the body that is coupled to the proximal LD1 side of the wire 2, thereby moving the stent 1 back and forth in the catheter or the blood vessel. As shown in FIGS. 9 to 14 described later, through pulling (moving back) or pushing (moving forward) the catheter C without moving the wire 2 (the stent 1), it is possible to: remove the stent 1 from the catheter C to expand the stent 1: or sheathe the stent again in the catheter C.

The stent 1 includes the first stent body 10 and the second stent body 20, each of which is a substantially cylindrical structure. The stent 1 is a stent having such a double-layer structure in which the second stent body 20 is inserted into the first stent body 10 in at least a region that expands a narrowed site of a living body lumen. Here, the region that expands a narrowed site of a living body lumen refers to a region substantially contributing to expansion of the narrowed site during use. This region corresponds to a region expanding in a substantially cylindrical shape in a diameter-expanded state shown in FIGS. 1 and 2 of the present embodiment. Moreover, the above-described "at least" means that in the above-described region that expands a narrowed site, the second stent body 20 is required to be inserted into the first stent body 10. Note that the region in which the second stent body 20 is inserted into the first stent body 10 is not limited to the above-described region that expands a narrowed site (in the example of the present embodiment, a region expanded in a substantially cylindrical shape). In the present embodiment, the second stent body 20 is inserted into the first stent body 10 immediately before the position where the first stent body 10 and the second stent body 20 are coupled to the wire 2.

The first stent body 10 and the second stent body 20 are not coupled to each other in the radial direction. Specifically, the first stent body 10 and the second stent body 20 are indirectly coupled to each other via the wire 2, but are not coupled to each other in the other region. Thus, the first stent body 10 and the second stent body 20 are independently and flexibly deformable. Also in a diameter-expanded state, the first stent body 10 and the second stent body 20 just closely contact to each other in the radial direction but are not coupled (connected) to each other in the radial direction. Thus, the first stent body 10 and the second stent body 20 are independently deformable, and do not restrict deformation of each other.

The proximal LD1 side of the first stent body 10, the proximal LD1 side of the second stent body 20, and the distal LD2 side of the wire 2 are directly connected to each other. Here, "directly connected" refers to, for example, a form in which the proximal LD1 side of the first stent body 10 and the distal LD2 side of the wire 2 are connected to each other and the proximal LD1 side of the second stent body 20 and the distal LD2 side of the wire 2 are connected to each other. In this case, a position where the proximal LD1 side of the first stent body 10 is connected to the wire 2 may be the same as or different from a position where the proximal LD1 side of the second stent body 20 is connected to the wire 2. In the present embodiment, the position where the proximal LD1 side of the first stent body 10 is connected to the wire 2 is provided on the proximal side with respect to the position where the proximal LD1 side of the second stent body 20 is connected to the wire 2.

Alternatively, the proximal LD1 side of the first stent body 10, the proximal LD1 side of the second stent body 20, and the distal LD2 side of the wire 2 may be indirectly connected to each other. Here, "indirectly connected" refers to, for example, a form in which the proximal LD1 side of the first stent body 10 and the distal LD2 side of the wire 2 are connected to each other and the proximal LD1 side of the first stent body 10 and the proximal LD1 side of the second stent body 20 are connected to each other (or a connection form in which the two stent bodies are connected in an inverse relationship). In this case, the proximal LD1 side of the second stent body 20 is indirectly connected to the wire 2 via the proximal LD1 side of the first stent body 10. Alternatively, there may be another form in which the proximal LD1 side of the first stent body 10 and the proximal LD1 side of the second stent body 20 are connected to each other via a pipe-like sleeve (not shown) inserted over the distal LD2 side of the wire 2. In this case, the proximal LD1 side of the first stent body 10 and the proximal LD1 side of the second stent body 20 are indirectly connected to the distal LD2 side of the wire 2 via the sleeve. As described above, in the present specification, "indirectly connected" includes a form in which one of the proximal LD1 side of the first stent body 10 and the proximal LD1 side of the second stent body 20 is directly connected to the wire 2, and the other stent body, which is not connected directly to the wire 2, is indirectly connected to the wire 2 via the stent body directly connected to the wire 2. Examples of a method for connecting the proximal side end portion of each stent body and the wire 2 to each other may include welding, UV bonding, and silver solder infiltration, but is not particularly limited as long as the connection method is used for general medical equipment.

As described later, the stent 1 of the first embodiment is produced in such a manner that the second stent body 20, which has a greater outer diameter than that of the first stent body 10 in a natural state, is inserted into the first stent body 10 in a diameter-narrowed state. Thus, the second stent body 20 constantly presses the first stent body 10 outward in the radial direction RD, so that the first stent body 10 and the second stent body 20 are firmly in tight contact with each other.

As shown in FIG. 1, the proximal LD1 side end portions of the first stent body 10 and the second stent body 20, which are tapered and narrowed, are connected to the wire 2. Thus, the second stent body 20 is restricted from moving in the axial direction relative to the first stent body 10.

In the first stent body 10, as shown in FIG. 3A, a plurality of outer cells (first cells) 12 surrounded by first struts 11 are arrayed in a circumferential direction OD. In the first stent body 10, the plurality of outer cells 12 are arrayed in the axial direction LD. That is, the first stent body 10 has such a pattern that the plurality of outer cells 12 are arrayed in the circumferential direction OD and the axial direction LD. An opening portion 13 is formed in the outer cell 12. Moreover, the outer cells 12 adjacent to each other in the circumferential direction OD are coupled to each other at an overlapping portion 14.

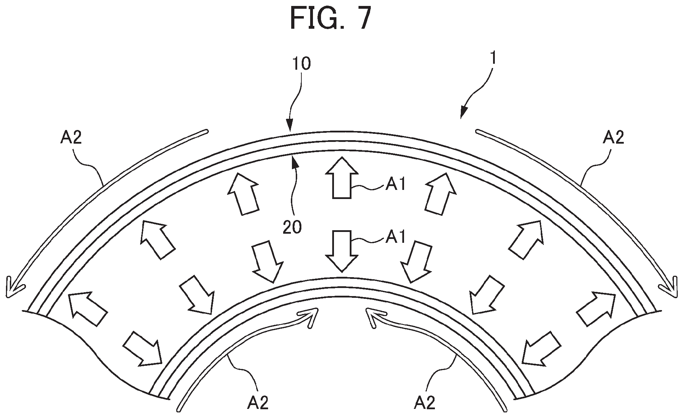

The overlapping portion 14 is a portion to which respective first struts 11 of four outer cells 12 adjacent to each other are connected. The overlapping portion 14 has a substantially rectangular shape elongated in the axial direction LD. The first struts 11 are individually connected to four corners of the overlapping portion 14. Each of the first struts 11 has a curved portion 15 which is formed at a portion where each of the first struts 11 is connected to the overlapping portion 14. Thus, when the expanded stent 1 is bent into a substantially U shape (see FIG. 7), each of the first struts 11 connected to the overlapping portion 14 is independently deformable. Therefore, the outer cells 12 arrayed in the circumferential direction OD can be more flexibly bent. As described above, the outer cells 12 arrayed in the circumferential direction OD can be flexibly bent, and therefore, the first stent body 10 is excellent in shape followability and diameter-narrowing performance.

In the second stent body 20, as shown in FIG. 3B, a plurality of inner cells (second cells) 22 surrounded by second struts 21 are arrayed in the circumferential direction OD. The plurality of inner cells 22 are continuously arrayed in the axial direction LD. That is, the second stent body 20 has such a pattern that the plurality of inner cells 22 are arrayed in the circumferential direction OD and the axial direction LD. The inner cells 22 adjacent to each other are coupled to each other at an overlapping portion 24.

The overlapping portion 24 is a portion to which the second struts 21 for the four inner cells 22 adjacent to each other are connected. The overlapping portion 24 has a substantially rectangular shape elongated in the axial direction LD. The second struts 21 are individually connected to four corners of the overlapping portion 24. Each of the second struts 21 has a curved portion 25 which is formed at a portion where each of the second struts 21 is connected to the overlapping portion 24. Thus, when the expanded stent 1 is bent into a substantially U shape, each of the second struts 21 connected to the overlapping portion 24 is independently deformable. Therefore, the inner cells 22 arrayed in the circumferential direction OD can be more flexibly bent. As described above, the inner cells 22 arrayed in the circumferential direction OD can be flexibly bent, and therefore, the second stent body 20 is excellent in shape followability and diameter-narrowing performance.

As shown in FIGS. 3A and 3B, in the stent 1 of the first embodiment, an outer cell 12 and an inner cell 22 have the same size, shape, and arrangement except for the distal LD2 side, as one example. That is, in the first embodiment, the patterns shown in FIG. 3A and FIG. 3B are substantially the same except for the distal LD2 side.

As shown in FIG. 3C, the first stent body 10 and the second stent body 20 are arranged to overlap with each other in a natural state such that the overlapping portion 24 between the adjacent inner cells 22 (the second stent body 20) is arranged in an opening portion 13 of the outer cell 12 (the first stent body 10). Specifically, one overlapping portion 24 between the adjacent inner cells 22 overlaps with one opening portion 13 of the outer cell 12. Thus, the density of the struts can be increased over the entire stent, and the surface area of the stent 1 can be increased. Since the overlapping portion 14 between the adjacent outer cells 12 has a configuration as shown in FIG. 3A, and the overlapping portion 24 between the adjacent inner cells 22 has a configuration as shown in FIG. 3B, the stent 1 of the present embodiment is excellent in shape followability and diameter-narrowing performance. Moreover, the stent 1, which is configured such that the outer cells 12 and the inner cells 22 have the configuration described above, produces effects that the narrowed stent 1 is easily sheathed in a catheter and the stent 1 expanded in a blood vessel is easily resheathed in a catheter.

In the first embodiment, a relationship between an outer diameter D1 of the first stent body 10 before assembly and an outer diameter D2 of the second stent body 20 before assembly is set to D1 < D2. Thus, as shown in FIG. 5, a second stent body 20A obtained by narrowing the second stent body 20 is inserted into the first stent body 10. As shown in FIG. 6, the second stent body 20 closely contacts the inside of the first stent body 10 in the circumferential direction OD due to self-expanding force of the second stent body 20 itself.

Thus, in the axial direction LD (see FIG. 1) of the stent 1, it is less likely that the positions of the first stent body 10 and the second stent body 20 are misaligned relative to each other. Note that for the sake of easy understanding, FIG. 5 shows only annular circumferential cell lines arrayed in the circumferential direction in each stent body, and for the sake of easy understanding of a tubular form, the number of cells is increased as compared with other figures.

In the first embodiment, the second stent body 20 inserted into the first stent body 10 in the diameter-narrowed state constantly presses the first stent body 10 outward in the radial direction RD. Consequently, even if the first stent body 10 and the second stent body 20 are not coupled to each other in the radial direction, the first stent body 10 and the second stent body 20 can closely contact each other. Moreover, since the first stent body 10 and the second stent body 20 are not coupled to each other in the radial direction in the stent 1, the first stent body 10 and the second stent body 20 are independently and flexibly deformable. Further, the stent 1 has the total expansive force created by the first stent body 10 and the second stent body 20. Thus, the stent 1 can have a greater ratio of the expansive force to the surface area than that of a stent having a single-layer structure.

The stent 1 (the first stent body 10, the second stent body 20) may be made of a material having a high biological compatibility. The stent 1 is preferably made of, but is not particularly limited to, a material having superelastic properties, such as nickel titanium (Ni-Ti) alloy. The first stent body 10 and the second stent body 20 may be produced, for example, in such a manner that substantially cylindrical tubes made of the above-described material are machined with a laser.

The stent 1 may be loaded with a medical agent such as a physiologically active substance (a medical agent for inhibiting intima thickening, etc.), and specifically, may carry the medical agent so as to dissolve out the medical agent in the lumen structure.

For example, the first stent body 10 and the second stent body 20 are produced by machining tubes having outer diameters of about 0.4 to 3 mm with a laser to form mesh patterns, and then, stretching the mesh patterns in the radial direction to expand to desired diameters. As described above, the second stent body 20 is inserted into the first stent body 10, and subsequently, the stent 1 having the double-layer structure can be produced. The stent 1 having the double-layer structure is sheathed in the catheter while being narrowed in the radial direction from the natural state. The stent 1, which is made of, e.g., an elastic material such as superelastic alloy or shape-memory alloy, expands in diameter due to the self-expanding force when the stent 1 sheathed in the catheter is pushed out.

Next, an effect in a case where the stent 1 of the first embodiment is removed after having been temporarily placed in a bent blood vessel will be described herein.

The stent 1 of the present embodiment is a stenosis dilation stent used to expand a lumen of a stenotic part in order to ensure the patency of a lesion area when stenosis occurs in a living body lumen. Therefore, the stent 1 contacts an inner wall of the living body lumen at a predetermined expansion force to be placed in the living body lumen. Thus, when a position of the stent 1 is accidentally moved while the stent 1 is in contact with the inner wall of the living body lumen, the inner wall of the living body lumen may be damaged. Consequently, it is important to operate the stent 1 so that a position of the stent 1 is not accidentally moved in a state in which a stent body is deployed. Since the first struts 11 and the second struts 21 are moved along with expanding and narrowing operations of the stent 1, the markers provided on the first struts 11 and the second struts 21 are suitable for checking the expanded or narrowed stent 1. On the other hand, the markers provided on the first struts 11 and the second struts 21 are not suitable for checking the movement of the entire stent 1. The wire 2 is connected to the stent 1, and therefore, a movement amount of the entire stent 1 coincides with a movement amount of the wire 2. In the present embodiment, the wire 2 is checked with a marker which is provided on the wire 2, thereby making it possible to check the movement of the entire stent 1.

The stent 1 of the first embodiment includes, as markers, a first wire marker (a first marker) 31, a second wire marker (a second marker) 32, a first stent marker 111, and a second stent marker 121. These markers each are a member serving as a mark for checking the position and the state of the stent 1 under observation by radiation in the tubular organ such as a blood vessel, and are formed of a material which is highly opaque to radiation (has a high radiopacity). Here, the radiation opacity indicates that, under observation using radiation during operation, the markers are more highly opaque to radiation than portions other than the markers in the stent 1 (other portions having no radiation opacity), and are opaque to radiation to the extent that the markers can be observed as images. The "radiation opacity" is required to obtain the "visibility" and "radiopacity" under observation using radiation.

First, descriptions will be given of configurations of conventional known markers that can be used as: the first wire marker 31, the second wire marker 32, the first stent marker 111, and the second stent marker 121 (hereinafter, simply referred to as markers) of the present embodiment; and various markers which will be described in the second embodiment.

As a highly radiopaque material, which can be used as the marker, a metal or a synthetic resin may be used, and examples of such a metal material include gold, tantalum, platinum, tungsten, iridium, and platinum tungsten, and an alloy containing these materials. The examples also include a polymer material having radiation opacity to which a radiopaque filler or the like is added. When the marker is formed of a linear member, it is possible, for example, to use a composite wire made of nickel titanium which includes coaxially core material made of the above-described metal material.

As a method of attaching the markers to the stent 1, it is possible to suitably employ a conventional known processing method which are used when the markers are attached to the stent, such as gold-tin solder, silver-tin solder, laser welding, mechanical pressing, bonding with resin.

Specifically, examples of forms that can be used as the markers of the present embodiment may include a "ring marker", a "coil marker", a "crimping marker", a "radiopaque wire marker", and a "radiopaque strut or radiopaque wire". Note that the above-described exemplary forms and the designations are exemplified by being classified and referred to as for purposes of convenience by the applicant, and conventional known various marker forms can be used.

The ring marker is, for example, a form in which a highly radiopaque marker component having a ring shape or a substantially cylindrical shape is fixed to a strut or the like by soldering, bonding, or the like.

The coil marker is a form in which a linear member which is made of highly radiopaque material is processed into a coil and wound and fixed around a strut or the like by soldering, bonding, or the like.

The crimping marker is, for example, a form in which a highly radiopaque marker component having a ring shape or a substantially cylindrical shape is fixed to a strut or the like by crimping. Note that when the crimping marker is used for the first wire marker 31 and the second wire marker 32, the eccentricity due to crimping may make it difficult to insert the wire into the catheter, or the wire portion with no slit makes it difficult to secure the strength. Therefore, it may be desirable to use the ring marker or the coil marker for the first wire marker 31 and the second wire marker 32.

The radiopaque wire marker is a form in which a highly radiopaque wire is used as a marker, and is placed along or wound around a strut or the like, and an end portion of the wire is fixed to the strut by soldering, bonding, or the like. The radiopaque wire marker is a form similar to the above-described coil marker, but is classified separately from the coil marker in that the wire is not wound at a density to the extent that it can be referred to as a coil.

The radiopaque strut or the radiopaque wire is a form in which the strut or wire itself is formed of a highly radiopaque material or a form in which a highly radiopaque material is tightly formed on the entire surface or a partial surface of the strut by plating, coating, or the like.

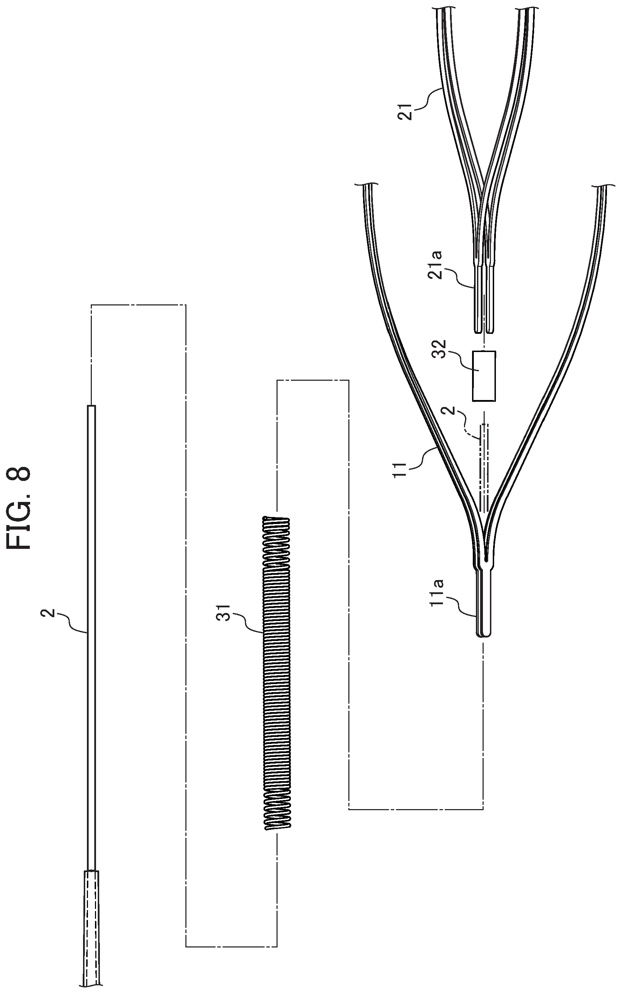

FIG. 8 is an exploded and enlarged diagram of the vicinity of a distal LD2 side end portion of the wire 2. In a proximal LD1 side end portion 11a of the first strut 11, two first struts 11 are arranged to face each other, and the distal LD2 side of the wire 2 is fixed by welding to a portion where the first struts 11 face each other in a state of being inserted therethrough.

The first wire marker 31 is a coil marker which is formed of a linear member of a highly radiopaque material into a coil shape, and is fixed by welding to a position covering the proximal LD1 side end portion 11a of the first strut 11. The first wire marker 31 is formed in a closely-wound coil shape around the center of the first wire marker 31, and is formed in an open-wound coil shape around both ends of the first wire marker 31. This configuration prevents bending of the wire 2.

At a proximal LD1 side end portion 21a of the second strut 21, two second struts 21 are arranged to face each other, and the distal LD2 side end portion of the wire 2 is located at a portion where the second struts 21 face each other and is fixed thereto by welding.

The second wire marker 32 is a ring marker in which a marker component, which is made of a highly radiopaque material and formed in a circular tube shape, is fixed to a strut by soldering. The second wire marker 32 is fixed by soldering to a position covering the proximal LD1 side end portion 21a of the second stent body 20. The second wire marker 32 is arranged to be separated toward a distal LD2 side from the first wire marker 31. A region between the first wire marker 31 and the second wire marker 32 is hereinafter referred to as a separation portion 33. A length of the separation portion 33 in the direction LD is preferably, for example, 0.6 mm or more and 2.0 mm or less.

The first wire marker 31 and the second wire marker 32 are offset in the axial direction LD and are arranged with a predetermined spacing. Such an arrangement makes it possible to correctly check the position of the proximal LD1 side end portion of each of the first stent body 10 and the second stent body 20. Moreover, the arrangement can serve as a mark for a pull-in position of the catheter C (see FIG. 9) in a diameter-expanded state as described later.

The first wire marker 31 is configured to have a longer length in the direction LD than the second wire marker 32. Therefore, the first wire marker 31 and the second wire marker 32 can be clearly distinguished and visually recognized. It is desirable to set a length of the first wire marker 31 to be 1.5 times or more as long as a length of the second wire marker 32 in the direction LD, such that it is easily distinguishable and visually recognizable both of the first wire marker 31 and the second wire marker 32. It is further desirable to set a length of the first wire marker 31 to be 2 times or more as long as a length of the second wire marker 32 in the direction LD. For example, when the length of the second wire marker 32 is 0.5 mm or more and 1.5 mm or less, it is desirable that a length of the first wire marker 31 in the direction LD is set to be 1.5 times or more as long as a length of the second wire marker 32 in the direction LD.

The first stent marker 111 is a crimping marker which is formed in a substantially square pipe shape, and is crimp-fixed to the distal LD2 side end portion of the first stent body 10. In the present embodiment, the first stent body 10 has two distal LD2 side end portions, and thus, two first stent markers 111 are provided.

The second stent marker 121 is a crimping marker which is formed in a substantially square pipe shape, and is crimp-fixed to the distal LD2 side end portion of the second stent body 20 in similar to the first stent marker 111. In the present embodiment, the second stent body 20 has two distal LD2 side end portions, and thus, two first stent markers 111 are provided.

In the observation by X-rays, it is difficult to visually distinguish and identify the first stent marker 111 from the second stent marker 121 using only a marker shape. In the present embodiment, the first stent marker 111 is arranged to be different positionally from the second stent marker 121 in the axial direction LD of the stent. More specifically, the second stent marker 121 is arranged closer on the distal LD2 side than the first stent marker 111. In the present embodiment, the inner cells 22 on the most distal LD2 side have a shape elongated on the distal LD2 side compared with the other cells (the inner cells 22 and the outer cells 12 on the more proximal LD1 side). In this manner, the second stent marker 121 is arranged closer on the distal LD2 side than the first stent marker 111.

With such a configuration, it is possible to determine whether the first stent marker or the second stent marker is found in the observation by X-rays when the first stent markers 111 and the second stent markers 121 are simultaneously observed. Accordingly, it is possible to visually distinguish and observe the first stent markers 111 and the second stent markers 121. Therefore, it is possible to correctly confirm not only the position of the distal LD2 side end portion of each of the first stent body 10 and the second stent body 20, but also how the diameter-expanded state or diameter-narrowed state occurs appropriately for each of the first stent body 10 and the second stent body 20.

Note that, in the present embodiment, the first stent markers 111 and the second stent markers 121 have the same shape and size. And the second stent markers 121 are arranged closer on the distal LD2 side than the first stent markers 111 in the axial direction LD of the stent 1. That is, the first stent markers 111 and the second stent markers 121 on the whole are configured to be different positionally from each other in the axial direction of the stent 1. The present disclosure is not limited thereto, and for example, the first stent markers 111 and the second stent markers 121 may in part be different positionally from each other in the axial direction of the stent 1. More specifically, for example, a length of a second stent marker 121 is configured to be longer than a length of a first stent marker 111 in the axial direction, so that either or both positions of the proximal LD1 side end portion and the distal LD2 side end portion of each of the first stent marker 111 and the second stent marker 121 may be different from each other. Moreover, all of the first stent markers 111 and the second stent markers 121 need not be as exemplified in the present embodiment. Alternatively, at least some of the first stent markers 111 may be arranged at the same positions as the second stent markers 121 in the axial direction LD. Similarly, some of the second stent markers 121 may be arranged at the same positions as the first stent markers 111 in the axial direction LD.

When the stent 1 of the first embodiment is deployed in a lesion area of a vascular lumen, it is possible to expand the vascular lumen, ensure the patency of the lesion area c, and restore blood flow accordingly. When the stent 1 is removed (retrieved) from the vascular lumen after a predetermined period of time, it is possible to prevent the occurrence of complication such as restenosis, reocclusion, and thrombosis caused by the indwelling stent. When the stent 1 is expanded or narrowed, the first stent markers 111 and the second stent markers 121 can be distinguished and recognized, and therefore, it is possible to adequately recognize the arrangement of the first stent body 10 and the second stent body 20. Accordingly, it is possible to more safely and reliably perform the operation. Since the stent 1 of the first embodiment has excellent shape followability, the vascular preservation is high. Note that the stent 1 of the first embodiment can be used not only for treatment of a blood vessel but also for treatment of lumen structures such as the esophagus and large intestine.

The stent 1 of the first embodiment can also be used for treatment of a cerebrovascular spasm. Note that stents of other embodiments to be described later also produce advantageous effects similar to those of the stent 1 of the first embodiment.

### (Method For Capturing Image)

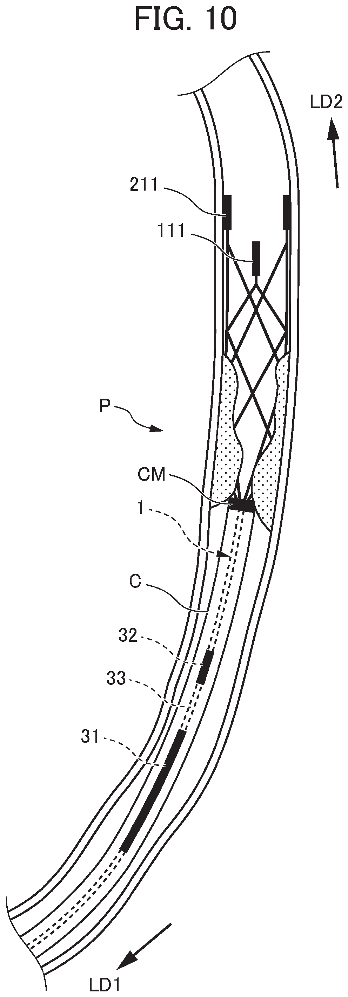

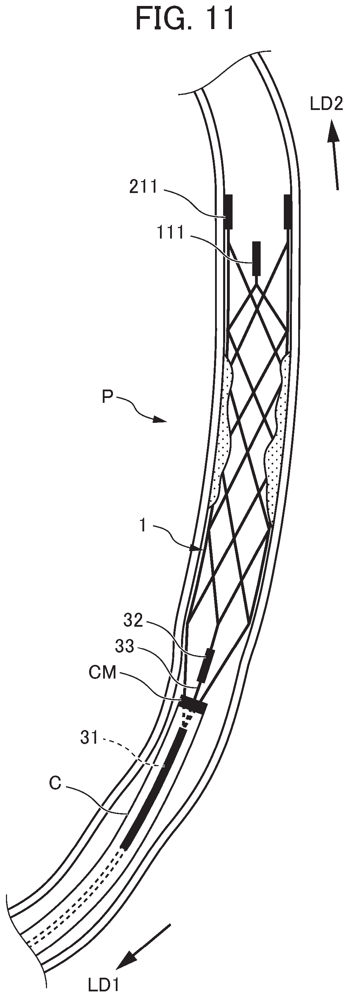

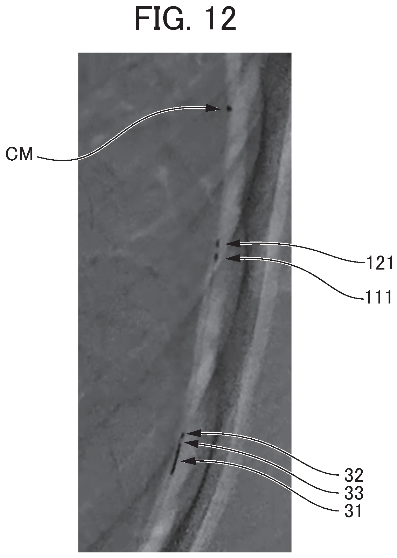

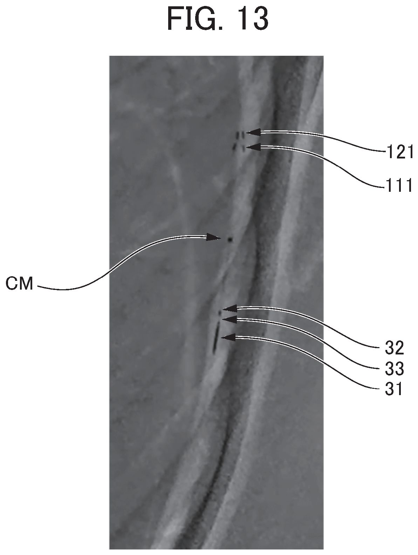

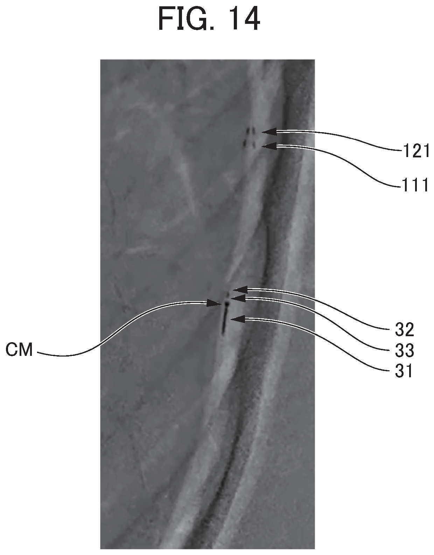

Next, to perform observation by radiation, a method for capturing an image of a stent of the present invention will be described. FIG. 9 is a diagram schematically showing an example of an image captured in capturing an image of the second stent marker 121 in a diameter-narrowed state of the stent 1 in a position where the stent 1 is to be expanded in the living body lumen. FIG. 10 is a diagram schematically showing an example of an image captured in capturing an image of the second stent marker 121 in an intermediate state while transitioning from a diameter-narrowed state to a diameter-expanded state of the stent 1 in the position where the stent 1 is to be expanded in the living body lumen. FIG. 11 is a diagram schematically showing an example of an image captured in capturing an image of the second stent marker 121 in a diameter-expanded state of the stent 1 in the position where the stent 1 is expanded in the living body lumen. FIG. 12 is a diagram showing an example of an image captured in capturing an image in a diameter-narrowed state of the stent. FIG. 13 is a diagram showing an example of an image captured in capturing an image while transitioning the stent. FIG. 14 is a diagram showing an example of an image captured in capturing an image in a diameter-expanded state of the stent.

The distal LD2 side of a catheter C includes a catheter marker CM which is highly opaque to X-rays so that the position can be visually recognized under observation by X-rays. The catheter marker CM is provided at the distal LD2 side end portion or in the vicinity of the distal LD2 side end portion of the catheter C. Note that the vicinity of the distal LD2 side of the distal end of the catheter C includes not only a position of a complete end portion but also a position distanced apart from the end portion within 1.5 mm. Note that in the examples shown in FIGS. 9 to 14, an offset amount by which the second stent marker 121 is arranged offset toward the distal LD2 side with respect to the first stent marker 111 is shown greater than that in FIG. 1 of the first embodiment.

When the blood vessel is expanded, the stent 1 sheathed in the catheter C in a state in which the stent is narrowed as shown in FIGS. 9 and 12 is delivered together with the catheter C through the blood vessel to a position P where the blood vessel is to be expanded. The position P where the blood vessel is to be expanded is, for example, a portion where an inner diameter of the blood vessel is smaller than other portions due to stenosis. At this time, a practitioner captures an X-ray transmission video of the first stent marker 111 and the second stent marker 121 using an X-ray imaging apparatus (capturing image in diameter-narrowed state of stent). The first stent marker 111 and the second stent marker 121 are arranged on the distal LD2 side of the stent 1 of the first embodiment, so that the relative position relationship between these markers and the catheter marker CM can be checked. Therefore, it is possible to visually check that the stent 1 is adequately sheathed in the catheter C and delivered to a desired position.

Next, when the catheter C is pulled toward the proximal LD1 side while preventing the wire 2 from accidentally moving in the direction LD to maintain the position of the stent 1 in the blood vessel, the stent 1 starts to expand from the distal LD2 side (see FIGS. 10 and 13). At this time, the practitioner captures an X-ray transmission video of the first stent marker 111 and the second stent marker 121, and the first wire marker 31 and the second wire marker 32 using an X-ray imaging apparatus (capturing an image while transitioning the stent). It is possible to check the positions of the first stent marker 111 and the second stent marker 121 relative to the catheter marker CM, and the positions of the first wire marker 31 and the second wire marker 32 relative to the catheter marker CM on the basis of the imaging results. Moreover, the second stent marker 121 is arranged closer on the distal LD2 side than the first stent marker 111, so that it is possible to distinguish and recognize the first stent marker 111 and the second stent marker 121. Therefore, it is possible to visually check that the expansion of the stent 1 adequately advances, the position of the stent 1 is not accidentally moved, and the expansion of the stent 1 is started.

When the expansion of the stent 1 is completed, the state shown in FIGS. 11 and 14 is achieved. At this time, a practitioner captures X-ray transmission videos of the first stent marker 111, the second stent marker 121, the first wire marker 31 and the second wire marker 32 using an X-ray imaging apparatus (capturing image in diameter-expanded state of stent). Similarly to the step of the capturing of an image during the transition of a stent, , it is possible to distinguish and recognize the first stent marker 111 and the second stent marker 121 in the step of capturing an image in the diameter-expanded state of the stent since the second stent marker 121 is arranged closer on the distal LD2 side than the first stent marker 111. The separation portion 33 is provided between the first wire marker 31 and the second wire marker 32, such that the markers are apart from each other by a predetermined distance. Moreover, when the catheter marker CM is located at a position overlapping with the separation portion 33 or at a position closer to the proximal LD1 side than the separation portion 33, it can be checked that the catheter C is reliably pulled from the range in which the stent 1 expands (the range of the first stent body and the second stent body). Consequently, when the capturing of an image in a diameter-expanded state of the stent is performed for a state in which the catheter marker CM is located at a position overlapping with the separation portion 33 or another position closer on the proximal LD1 side than the separation portion 33, it is possible to visually confirm that the expansion of the stent 1 has been adequately completed at an appropriate position.

Note that when the expanded stent 1 is resheathed in the catheter C, the above-described operation and capturing of an image are executed in reverse order. Also in any capturing an image when the expanded stent 1 is resheathed in the catheter C, an operation of pushing the catheter C toward the distal LD2 side is performed while preventing the wire 2 from accidentally moving in the direction LD. It is possible to easily and reliably determine whether the wire 2 is in motion by observing the first wire marker 31, the second wire marker 32, and the separation portion 33.

As described above, according to the first embodiment, the stent 1 is configured to include the first wire marker 31 and the second wire marker 32 which are spaced apart with the separation portion 33 at a predetermined distance. In addition, the length of the separation portion 33 is different from the distance between the first stent marker 111 and the second stent marker 121. Moreover, the length of the first wire marker 31 is longer than the length of the second wire marker 32. And the separation portion 33 is provided between the markers having different lengths. Accordingly, the visually recognized arrangement of markers is. Therefore, the first wire marker 31 and the second wire marker 32 are distinguishable from the first stent marker 111 and the second stent marker 121. Similarly, the first wire marker 31 and the second wire marker 32 are provided to be separated at a predetermined distance, so that they are distinguishable from the catheter marker CM. When transitioning from the diameter-narrowed state to the diameter-expanded state, the catheter marker CM is moved to the position overlapping with the separation portion 33, so that it is possible to check that the catheter C is moved to the position where the stent can be sufficiently expanded. The case is assumed where when the catheter marker CM overlaps with the first wire marker 31 or the second wire marker 32, the first wire marker 31 and the second wire marker 32 are hardly distinguishable from each other. However, even in such a case, since the separation portion 33 is provided, it can be easily determined that the catheter marker CM is located at a position overlapping with the separation portion 33. Further, the length of the first wire marker 31 is configured to be longer than the length of the second wire marker 32 in the direction LD. Therefore, the first wire marker 31 and the second wire marker 32 can be clearly distinguished and visually recognized. Even in a case of a repeatedly bending blood vessel, for example, the orientations of the proximal side and the distal side can be easily determined.

### (Second Embodiment)

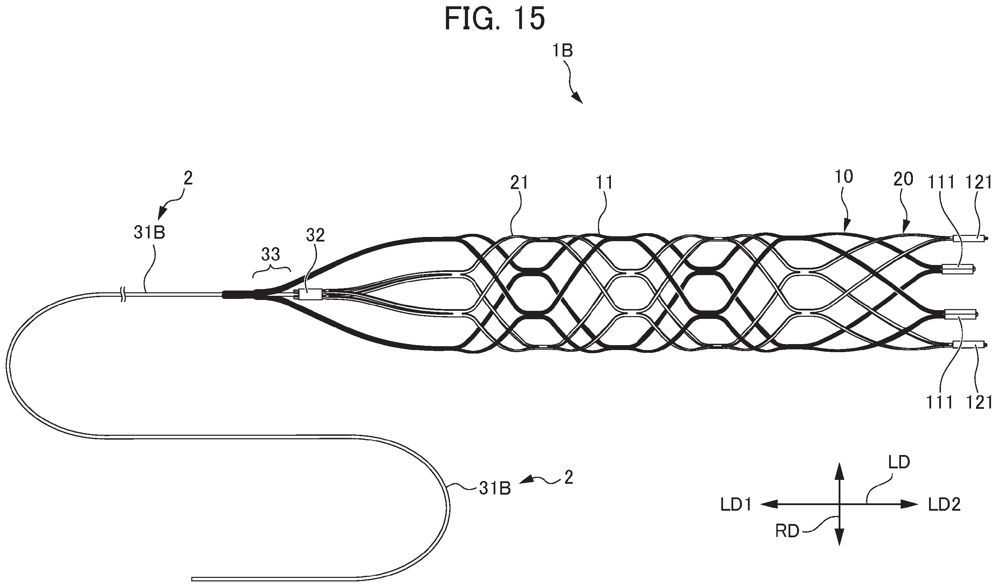

FIG. 15 is a schematic side view of a stent 1B of a second embodiment. The stent 1B of the second embodiment has a similar configuration to the stent 1 of the first embodiment except that the form of a first wire marker 31B is different from that of the first wire marker 31 of the first embodiment. Thus, the same reference numerals are used as elements having functions similar to those of the above-described first embodiment, and overlapping description thereof will be omitted as necessary.

The first wire marker 31B is configured to be a highly radiopaque material which is tightly formed on an area required for the surface of a wire 2 by plating, coating, or the like. Note that the first wire marker 31B may be formed as a coil marker similarly to the first embodiment or may be formed as a marker having another form.

A distal end position on the distal LD2 side of the first wire marker 31B of the second embodiment is provided at a position where a separation portion 33 is provided between the first wire marker 31B and a second wire marker 32 similarly to the first wire marker 31 of the first embodiment. A rear end position on the proximal LD1 side of the first wire marker 31B is continuously provided up to a rear end position on the proximal LD1 side of the wire 2. Therefore, the stent 1B of the second embodiment can be visually recognized up to the rear end position on the proximal LD1 side of the wire 2 under observation using radiation. Such a configuration makes it possible to quickly find whether a force that pushes or pulls the stent 1B is exerted on the wire 2. This will be described below using schematic diagrams.

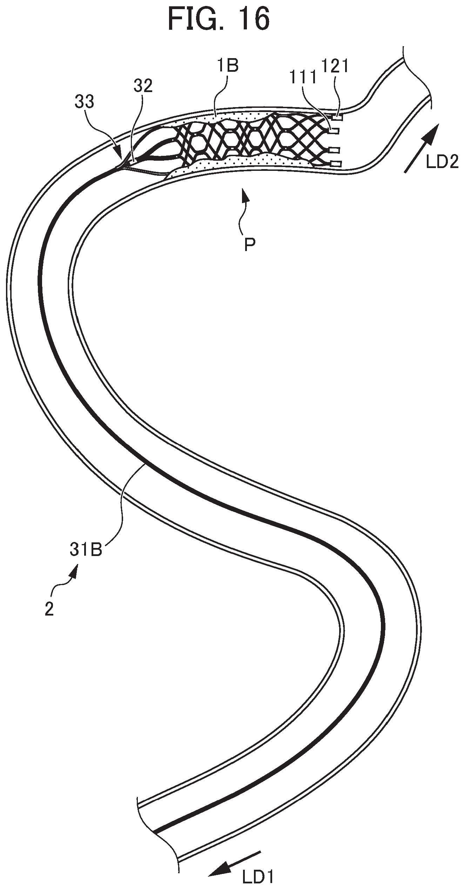

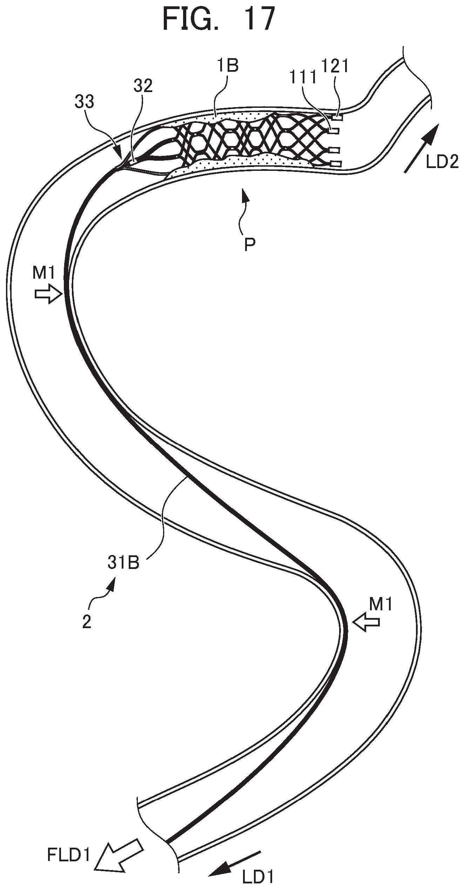

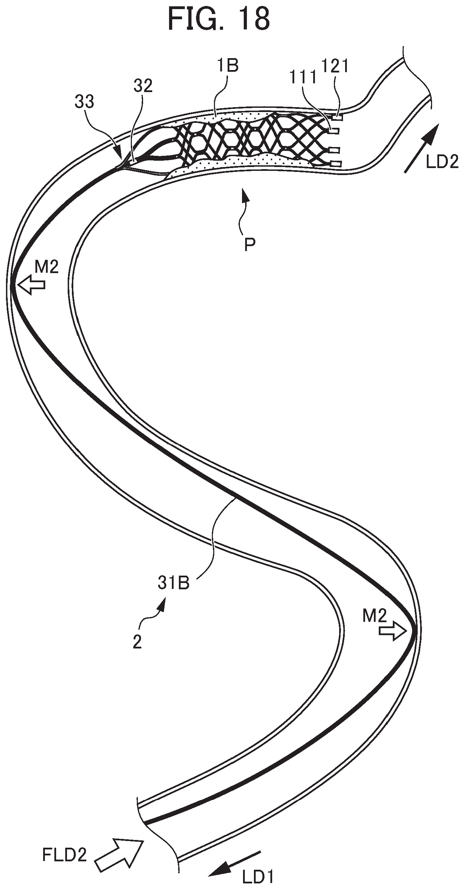

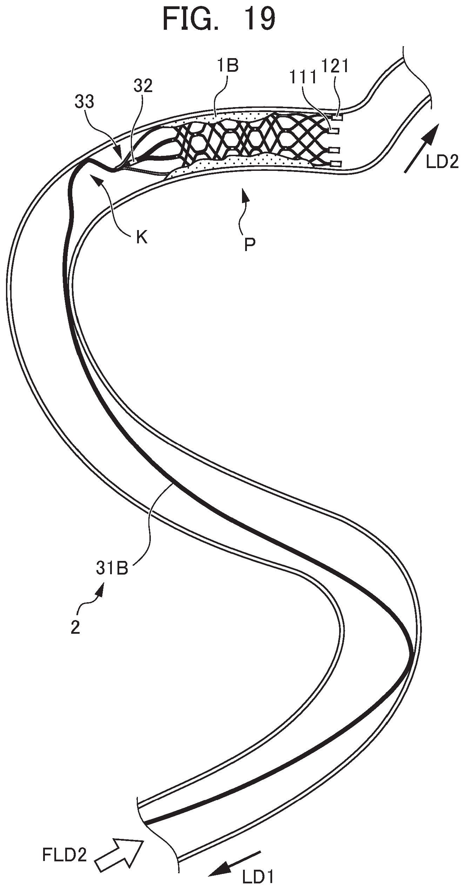

FIG. 16 is a schematic diagram showing an ideal state in which an extra force is not applied to the wire 2 in a state in which the stent 1B of the second embodiment is expanded. FIG. 17 is a schematic diagram showing a state in which a force with which the wire 2 is pulled toward a proximal LD1 side is applied in a state in which the stent 1B of the second embodiment is expanded. FIG. 18 is a schematic diagram showing a state in which a force with which the wire 2 is pushed toward a distal LD2 side is applied in a state in which the stent 1B of the second embodiment is expanded. FIG. 19 is a schematic diagram showing a state in which a force with which the wire 2 is pushed toward a distal LD2 side is applied in a state in which the stent 1B of the second embodiment is expanded. Note that in FIGS. 16 to 19, the catheter is not shown for simplicity of viewing the state of the wire 2.

Similarly to the stent 1 of the first embodiment, the stent 1B of the second embodiment is a stenosis dilation stent used to expand a lumen of a stenotic part in order to ensure the patency of a lesion area when stenosis occurs in a living body lumen. Thus, when a position of the stent 1B is accidentally moved while the stent 1B is in contact with the inner wall of the living body lumen, the inner wall of the living body lumen may be damaged. Consequently, it is important to operate the stent 1B so that a position of the stent 1B is not accidentally moved in a state in which a stent body is deployed.

When a force FLD1 with which the wire 2 is pulled toward the proximal LD1 side is applied in a state in which the stent 1B is expanded, the wire 2 is, for example, moved in a direction (an arrow M1 in FIG. 17) approaching the inner wall of the inwardly curved blood vessel, and the state in FIG. 16 is changed to the state in FIG. 17. This change is characteristic when the force FLD1 with which the wire 2 is pulled toward the proximal LD1 side is applied. Therefore, under observation using radiation, observing the state of the wire 2 (the first wire marker 31B) allows to quickly know that the force FLD1 with which the wire 2 is pulled toward the proximal LD1 side is applied.

When a force FLD2 with which the wire 2 is pushed toward the distal LD2 side is applied in a state in which the stent 1B is expanded, the wire 2 is, for example, moved in a direction (an arrow M2 in FIG. 18) approaching the inner wall of the outwardly curved blood vessel, and the state in FIG. 17 is changed to the state in FIG. 18. Alternatively, as shown in FIG. 19, the case is also assumed where a part of the wire 2 (a portion of an arrow K in FIG. 19) is largely bent. Such a change is a characteristic change when the force FLD2 with which the wire 2 is pushed toward the distal LD2 side is applied. Therefore, under observation using radiation, observing the state of the wire 2 (the first wire marker 31B)allows to quickly know that the force FLD2 with which the wire 2 is pushed toward the distal LD2 side is applied.

Note that, in order to quickly detect the above-described forces applied to the wire 2, the first wire marker 31B need not be provided up to the proximal LD1 side end portion of the wire 2. For example, it is only required that the first wire marker 31B is provided in a range of 20 mm or more toward the proximal LD1 side of the wire 2 from the distal LD2 side end portion at which a separation portion 33 is provided between the first wire marker 31B and the second wire marker 32. Alternatively, for example, the first wire marker 31B may be provided in a range of 200 mm or less toward the proximal LD1 side of the wire 2 from the distal LD2 side end portion at which the separation portion 33 is provided between the first wire marker 31B and the second wire marker 32.

As described above, according to the second embodiment, the first wire marker 31B is provided in a range of 20 mm or more toward the proximal LD1 side of the wire 2 from the distal LD2 side end portion at which the separation portion 33 is provided between the first wire marker 31B and the second wire marker 32. Therefore, it is possible to quickly detect that a force with which the wire 2 is pulled or pushed is applied. Consequently, a first stent body 10 and a second stent body 20 can be prevented from accidentally moving while rubbing against the inner wall of the blood vessel or the like, and safer operation can be performed, accordingly.

### (Modification)

The present disclosure is not limited to the above-described embodiments and various modifications and changes can be made, and these modifications and changes are also included in the scope of the present disclosure.
(1) In each embodiment, a specific form of a marker has been described as an example. However, the specific form of the marker in each embodiment is not limited to the example shown in the embodiment, and conventionally known markers of various forms may be available. For example, a portion of the ring marker may be replaced with the coil marker, and a portion of the radiopaque wire may be replaced with the coil marker.
(2) In each embodiment, the description has been made in which the marker exists also on the strut portion. The present invention is not limited thereto, and for example, the marker on the strut portion may be omitted.
(3) In each embodiment, the description has been made in which the stent has a double-layer structure as an example. The present invention is not limited thereto, and for example, the stent may have a single-layer structure.
(4) In the second embodiment, the description has been made in which the rear end position on the proximal LD1 side of the first wire marker 31B is continuously provided up to a rear end position on the proximal LD1 side of the wire 2, as an example. The present invention is not limited thereto, and for example, the first wire marker 31B may be discontinuously provided. Here, discontinuously refers to, for example, a form in which a plurality of the first wire markers 31B are arrayed at intervals in a form of broken lines.

Note that each embodiment and modification may be combined as necessary, but detailed description thereof will be omitted. The present disclosure is not limited to each embodiment described above.

### EXPLANATION OF REFERENCE NUMERALS

1, 1B Stent
2 Wire
10 First stent body
11 First strut
11a End portion
12 Outer cell
13 Opening portion
14 Overlapping portion
15 Curved portion
20, 20A Second stent body
21 Second strut
21a End portion
22 Inner cell
23 Opening portion
24 Overlapping portion
25 Curved portion
31, 31B First wire marker
32 Second wire marker
33 Separation portion
111 First stent marker
121 Second stent marker
C Catheter
CM Catheter marker

## Claims

1. A stent that is inserted into and delivered through a catheter and is used for an application to expand a stenotic part in a living body lumen, the stent comprising:
a wire;
a stent body that is connected to a distal side of the wire;
a first wire marker provided on the wire; and
a second wire marker provided on the wire to be distanced from the first wire marker toward a distal side in an axial direction of the stent.

2. A stent that is inserted into and delivered through a catheter and is used for an application to expand a stenotic part in a living body lumen, the stent comprising:
a wire;
a first stent body that is connected to a distal side of the wire and in which a plurality of first cells including a plurality of first struts are arrayed in a circumferential direction and in a central axis direction;
a second stent body in which a plurality of second cells including a plurality of second struts are arrayed in the circumferential direction and the central axis direction and at least a region that expands the living body lumen is inserted into the first stent body;
a first wire marker provided on the wire; and
a second wire marker provided on the wire to be distanced from the first wire marker toward a distal side in an axial direction of the stent.

3. The stent according to claim 1 or 2, wherein
the first wire marker is configured to have a longer length than the second wire marker.

4. The stent according to claim 3, wherein
the first wire marker is provided toward a proximal side of the wire continuously or in multiple parts that are distanced apart from each other, in a range of 20 mm or more from a position of a distal side end portion of the first wire marker toward a proximal side.

5. A method for capturing an image of the stent according to claim 1 or 2 in a living body lumen, the method comprising:
capturing an image of the first wire marker and the second wire marker in a diameter-narrowed state of the stent at a position where the stent is to be expanded in the living body lumen; and
capturing an image of the first wire marker and the second wire marker in a diameter-expanded state of the stent.

6. The method for capturing an image of the stent according to claim 5, wherein
a catheter marker which is highly opaque to radiation is provided at a distal end of the catheter or in a vicinity of the distal end,
in capturing the image in the diameter-narrowed state of the stent, an image of the catheter marker is captured together with the first wire marker and the second wire marker in a state in which the catheter marker is on the distal side with respect to the second wire marker, and
in capturing the image in the diameter-expanded state of the stent, an image of the catheter marker is captured together with the first wire marker and the second wire marker in a state in which the catheter marker overlaps with a separation portion located between the first wire marker and the second wire marker or in a state in which the catheter marker is on a proximal side with respect to the separation portion.

7. The method for capturing an image of the stent according to claim 6, wherein
when the stent is removed from the catheter and expanded, after capturing the image in the diameter-narrowed state of the stent is performed, the catheter is moved toward the proximal side while maintaining a position of the stent, and capturing the image in the diameter-expanded state of the stent is performed, and
then, when the stent is sheathed in the catheter, the catheter is moved toward the distal side while maintaining the position of the stent and capturing an image in the diameter-narrowed state of the stent is performed again.
